# EUROPEAN PATENT APPLICATION

(11) **EP 2 821 107 A1**
(43) Date of publication of application: **07.01.2015**
(21) Application number: 13175266.9
(22) Date of filing: 05.07.2013
(51) Int. Cl.: A61P 35/00, A61K 36/07, A61K 36/71

(54) **Composition and use thereof for the treatment of benign and malign diseases**

(71) Applicant: Cellquantum AG, 6004 Luzern (CH)
(72) Inventor: Temper, Rupert, 4342 Baumgartenberg (AT)
(74) Representative: Vos, Derk

(57) **Abstract**

The present disclosure relates to a composition comprising several plants, preferably extracts, useful in the treatment and prevention of tumor indications, the use of such composition for the treatment or prevention of tumor indications, and a method for the production of such composition. The composition comprises the compounds *agaricus subrufescens, pineapple* and *ranunculus.*

## Description

### BACKGROUND OF THE INVENTION

### Field of the invention

The present disclosure relates to a composition comprising several plants, preferably extracts, useful in the treatment and prevention of tumor indications, the use of such composition for the treatment or prevention of tumor indications, and a method for the production of such composition.

### Background of the invention

Cancer, such as tumor indications, also referred to as tumors, is still one of the most threatening diseases. This is also partly due to the difficulty modem medicine still has in curing cancer.

Many different strategies to cure cancer are known. However, the newly developed drugs are usually limited in their applicability and often have severe side effects.

There is thus still a need for a drug that is useful in the treatment or prevention of cancer, especially tumor indications, without severe side effects.

### SUMMARY OF THE INVENTION

In a first aspect, the present disclosure provides a composition as disclosed in claim 1.

In another aspect, the present disclosure provides the use of a composition of the present disclosure in the treatment or prevention of tumor indications, preferably tumors to the genitourinary system, with or without metastases, breast or mamma carcinoma, with or without metastases, cancer in the gynecologic system, with or without metastases, lung cancer, with or without metastases, tumors to the brain, colorectal cancer, with or without metastases, pancreatic cancer, with or without metastases, parathyroid carcinoma, with or without metastases, cancer in the digestive system, with or without metastases, hepatic cancer, with or without metastases, melanoma, with or without metastases, leukemia, and/or benign tumor indications.

In another aspect, the present disclosure provides a method for the treatment or prevention of a tumor indication in an animal, comprising administering to an animal in need thereof, an effective amount of a composition of the present disclosure.

In another aspect, the present disclosure provides the use of a composition of the present disclosure for the preparation of a medicament, preferably for the treatment or prevention of a tumor indication.

In still another aspect, the present disclosure provides a method for the preparation of a composition of the present disclosure.

### DETAILED DESCRIPTION OF THE INVENTION

The compositions of the first aspect of the present invention comprise the compounds *agaricus subrufescens, pineapple* and *ranunculus.*

In a preferred embodiment, the composition additionally comprises the compound *cyanobacteria,* preferably *cyanobacteria spirulina.*

In another preferred embodiment, the composition additionally comprises at least one compound selected from the group consisting of *carica papaya,* preferably green *Carica papaya;* and *sambucus,* preferably *sambucus flos.*

In another preferred embodiment, the *ranunculus* is dried *ranunculus acris* or *ranunculus repens.*

In another preferred embodiment, the composition comprises at least the compounds *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos.*

In another preferred embodiment, the composition comprises the compound *nicotiana.* It is further preferred that the composition additionally comprises, apart from *nicotiana,* also *petroselium* and magnesium phosphoricum, or *hypericum perforatum* and magnesium phosphoricum.

In another preferred embodiment, the composition comprises at least the compounds *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos; hypericum perforatum; petroselium; nicotiana;* and magnesium phosphoricum.

In another preferred embodiment, the compound that is contained in the composition is a plant or algae, preferably the compound is an extract of said plant or algae, respectively, and more preferably an aqueous extract.

In another preferred embodiment, any of the compounds selected from the group consisting of *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos; hypericum perforatum; petroselium; nicotiana;* and magnesium phosphoricum, is present as an extract, preferably an aqueous extract, in an amount of from 50 to 300 ml, preferably from 70 to 260 ml, based on one liter of composition.

In another preferred embodiment, any of the compounds selected from the group consisting of *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos; hypericum perforatum; petroselium; nicotiana;* and magnesium phosphoricum, is present as an extract, preferably an aqueous extract.

In another preferred embodiment, the composition further comprises a sugar, preferably sucrose.

In another preferred embodiment, the composition further comprises oxygen (O₂), preferably containing oxygen (O₂) in an amount of more than 23 mg/l.

In another preferred embodiment, the composition further comprises calcium phosphate, potassium citrate, silicon dioxide, sodium chloride, magnesium citrate, lithium carbonate, zinc gluconate, hypericum perforatum, and cyanobacteria or agaricus subrufescens.

In another preferred embodiment, the composition further comprises colloidal silver.

In another preferred embodiment, the composition further comprises vitamins and/or minerals.

In another preferred embodiment, the composition is an aqueous composition.

The compositions of the present disclosure may be used for the treatment or prevention of a benign and malign tumor indication. In other words, the compositions of the present disclosure are useful in the treatment or prevention of a benign and malign tumor indication.

In a preferred embodiment, the benign and malign tumor indication is selected from the group consisting of
K1. Cancer to the genitourinary system with or without metastases;
K2. Mamma carcinoma with or without metastases;
K3. Cancer to the gynecologic system with or without metastases;
K4. Lung Cancer with or without metastases;
K5. Tumors to the brain;
K6. Colorectal cancer with or without metastases;
K7. Pancreatic cancer with or without metastases;
K8. Parathyroid carcinoma with or without metastases;
K9. Cancer to the digestive system with or without metastases;
K10. Hepatic Cancer with or without metastases;
K11. Melanoma;
K12. Leukemia; and
K13. Benign tumor indications.

In another preferred embodiment, the composition is administered orally, intravenously, subcutaneously, or topically, preferably orally or intravenously, and more preferably orally, when used in the treatment or prevention of a benign and malign tumor indication.

In another preferred embodiment, the composition is administered once daily up to 10 times daily, preferably once daily up to five times daily, and more preferably three times daily.

In still another preferred embodiment, the composition treatment is applied for at least four weeks, preferably at least 12 weeks, and more preferably between 12 weeks and 48 weeks, when the composition is used in the treatment or prevention of a benign and malign tumor indication.

In another preferred embodiment, the composition is co-administered together with at least one additive, which is not necessarily taken at the same time and/or mixed with the composition.

In a further preferred embodiment, the at least one additive is selected from the group consisting of
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
(b) colloidal silver;
(c) oxygen water, preferably containing oxygen (O₂) in an amount of more than 23 mg/l;
(d) vitamin(s); and
(e) mineral(s).

In another further preferred embodiment, the composition is co-administered together with at least the additive
(a) composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof.

In another further preferred embodiment, the composition is co-administered together with at least the additive
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof; and
(b) colloidal silver.

In another further preferred embodiment, the composition is co-administered together with at least the additives
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof; and
(c) oxygen water, preferably containing oxygen (O₂) in an amount of more than 23
   mg/l.

In another further preferred embodiment, the composition is co-administered together with at least the additives
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
(b) colloidal silver; and
(c) oxygen water, preferably containing oxygen (O₂) in an amount of more than 23 mg/l.

In another preferred embodiment, the composition Q4 comprises a mixture of calcium phosphate, potassium citrate, silicon dioxide, sodium chloride, magnesium citrate, lithium carbonate, zinc gluconate, hypericum perforatum, and cyanobacteria or agaricus subrufescens.

The present disclosure is also directed to a composition as disclosed in the present disclosure for use in the treatment or prevention of a benign and malign tumor indication. The preferred benign and malign tumor indications are listed herein.

The present disclosure is also directed to a method for the treatment or prevention of a benign and malign tumor indication in an animal, comprising administering to an animal in need thereof, an effective amount of a composition as disclosed herein. The preferred benign and malign tumor indications are listed herein.

The present disclosure is also directed to the use of a composition as disclosed in the present disclosure for the preparation of a medicament. In a preferred embodiment, the medicament is a medicament for the treatment or prevention of a benign and malign tumor indication. The preferred benign and malign tumor indications are listed herein.

Furthermore, the present disclosure is also directed to a method for the preparation of a composition as disclosed herein, wherein the composition is an aqueous composition, comprising the steps of
(a) providing ready to use aqueous extracts of *agaricus subrufescens, pineapple* and *ranunculus,* and admixing said extracts to form a mixture;
(b) adding sucrose to said mixture; and
(c) optionally enriching said mixture with oxygen.

Finally, the present disclosure is also directed to a kit of part comprising
- a composition as disclosed in the present disclosure; and
- an additive comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and a combination thereof; and/or
- oxygen water, preferably containing oxygen (O₂) in an amount of more than 23 mg/l; and/or
- colloidal silver; and/or
- vitamin(s); and/or
- mineral(s); and
- optionally instructions for a dosage regimen.

It is understood that any of the preferred embodiments or compositions may be combined with each other resulting in further preferred embodiments of the present invention.

The compositions of the present disclosure are useful in the treatment of some specific types of tumor indications. Some of the more specific types of tumor indication are illustrated in the following in more detail.

### Cancer to the genitourinary system

The compositions of the present disclosure are useful in the treatment or prevention of cancer to the genitourinary system, with or without metastases.

Various types of cancer in the genitourinary system may be treated or prevented by the compositions of the present disclosure. Exemplary types of cancer in the genitourinary system include prostate or prostatic cancer, with or without metastases, testicular cancer, i.e., cancer at the testis, with or without metastases, urinary bladder cancer, with or without metastases, and renal cancer, with or without metastases.

*Prostate cancer* is a form of cancer that develops in the prostate, a gland in the male reproductive system. Most prostate cancers are slow growing; however, there are cases of aggressive prostate cancers. The cancer cells may metastasize (spread) from the prostate to other parts of the body, particularly the bones and lymph nodes. Conventionally, curative treatment generally involves surgery, various forms of radiation therapy, or, less commonly, cryosurgery; hormonal therapy and chemotherapy are generally reserved for cases of advanced disease (although hormonal therapy may be given with radiation in some cases).

*Testicular cancer* is a type of cancer that develops in the testicles, a part of the male reproductive system. It is the most common cancer in male aged 20-39 years. Since testicular cancers can spread, patients are conventionally usually offered adjuvant treatment - in the form of chemotherapy or radiotherapy - after surgery to remove the affected testicle (orchiectomy).

*Bladder cancer* is any of several types of malignancy arising from the epithelial lining of the urinary bladder. The most common type of bladder cancer recapitulates the normal histology of the urothelium and is known as transitional cell carcinoma. Bladder cancer characteristically causes blood in the urine.

*Renal cancer,* or kidney cancer, is a type of cancer that starts in the cells in the kidney. The two most common types of kidney cancer are renal cell carcinoma (RCC) and urothelial cell carcinoma (UCC) of the renal pelvis.

### Breast or mamma carcinoma

The compositions of the present disclosure are also useful in the treatment or prevention of breast or mamma carcinoma, with or without metastases.

*Breast cancer* is a type of cancer originating from breast tissue, most commonly from the inner lining of milk ducts or the lobules that supply the ducts with milk. The first noticeable symptom of breast cancer is typically a lump that feels different from the rest of the breast tissue. More than 80% of breast cancer cases are discovered when the woman feels a lump. Lumps found in lymph nodes located in the armpits can also indicate breast cancer.

### Cancer to the gynecologic system

Various types of cancer in the gynecologic system may be treated or prevented by the compositions of the present disclosure. Exemplary types of cancer in the gynecologic system include uterine cancer (in particular cancer to the cervix uteri), with or without metastases, and ovarian cancer, with or without metastases.

*Cervical cancer,* or cancer to the cervix uteri, arises from the transformation zone of the cervix, the lower portion of the uterus and connects to the upper aspect of the vagina.

*Ovarian cancer* is a cancerous growth arising from the ovary. Most (more than 90%) ovarian cancers are classified as "epithelial" and are believed to arise from the surface (epithelium) of the ovary.

### Lung Cancer

Also lung cancer, with or without metastases, may be treated or prevented by the compositions of the present disclosure.

*Lung cancer* is a disease characterized by uncontrolled cell growth in tissues of the lung. If left untreated this growth can spread beyond the lung in a process called metastasis into nearby tissue and eventually into other parts of the body. The main types of lung cancer are small-cell lung carcinoma (SCLC), also called oat cell cancer, and non-small-cell lung carcinoma (NSCLC). NSCLC is sometimes treated with surgery, whereas SCLC usually responds better to chemotherapy and radiotherapy.

Survival depends on stage, overall health, and other factors. Overall, 15% of people in the United States diagnosed with lung cancer survive five years after the diagnosis. Worldwide, lung cancer is the most common cause of cancer-related death in men and women, and is responsible for 1.38 million deaths annually, as of 2008.

### Tumors to the brain

Various types of cancer in the brain may be treated or prevented by the compositions of the present disclosure. Exemplary types of cancer to the brain include glioblastoma with or without metastases, anaplastic astrocytoma, and meningioma.

*Glioblastoma multiforme* (GBM) is the most common and most aggressive malignant primary brain tumor in humans, involving glial cells and accounting for 52% of all functional tissue brain tumor cases and 20% of all intracranial tumors. Despite being the most prevalent form of primary brain tumor, GBM incidence is only 2-3 cases per 100,000 people in Europe and North America. Conventional treatment can involve chemotherapy, radiation, radiosurgery, corticosteroids, antiangiogenic therapy, surgery and experimental approaches such as gene transfer.

With the exception of the brainstem gliomas, glioblastoma has the worst prognosis of any central nervous system (CNS) malignancy, despite multimodality treatment consisting of open craniotomy with surgical resection of as much of the tumor as possible, followed by concurrent or sequential chemoradiotherapy, antiangiogenic therapy with bevacizumab, gamma knife radiosurgery, and symptomatic management with corticosteroids.

*Anaplastic astrocytoma* is a WHO grade 3 type of astrocytoma. The growth rate and mean interval between onset of symptoms and diagnosis is approximately 1.5-2 years but is highly variable, being intermediate between that of low-grade astrocytomas and glioblastomas. Seizures are less common among patients with anaplastic astrocytomas compared to low-grade lesions.

*Meningiomas* are a diverse set of tumors arising from the meninges, the membranous layers surrounding the central nervous system. According to the American Brain Tumor Association, meningiomas are the most common primary brain tumor, representing one-third of all such tumors. These tumors are usually benign in nature; however, a small percentage is malignant. Symptomatic meningiomas are typically treated with either radiosurgery or conventional surgery.

### Colorectal cancer

The compositions of the present disclosure are also useful in the treatment or prevention of colorectal cancer, with or without metastases.

*Colorectal cancer,* commonly known as colon cancer or bowel cancer, is a cancer from uncontrolled cell growth in the colon or rectum (parts of the large intestine), or in the appendix. Cancers that are confined within the wall of the colon are often curable with surgery while cancer that has spread widely around the body is usually not curable and management then focuses on extending the person's life via chemotherapy and improving quality of life. Colorectal cancer is the third most commonly diagnosed cancer in the world, but it is more common in developed countries.

### Pancreatic cancer

The compositions of the present disclosure are also useful in the treatment or prevention of pancreatic cancer, with or without metastases.

*Pancreatic cancer* is a malignant neoplasm originating from transformed cells arising in tissues forming the pancreas. The most common type of pancreatic cancer, accounting for 95% of these tumors, is adenocarcinoma. Pancreatic cancer has a poor prognosis: for all stages combined, the 1- and 5-year relative survival rates are 25% and 6%, respectively; for local disease the 5-year survival is approximately 20% while the median survival for locally advanced and for metastatic disease, which collectively represent over 80% of individuals, is about 10 and 6 months respectively.

### Parathyroid carcinoma

The compositions of the present disclosure are also useful in the treatment or prevention of parathyroid carcinoma, with or without metastases. *Parathyroid carcinoma* is a rare malignant neoplasm resulting in parathyroid adenoma to carcinoma progression. It forms in tissues of one or more of the parathyroid glands and it is a rare cause of hypercalcemia.

### Cancer to the digestive system

Various types of cancer in the digestive system may be treated or prevented by the compositions of the present disclosure. Exemplary types of cancer in the digestive system include oesophageal cancer, with or without metastases, and gastric cancer, with or without metastases.

*Oesophageal cancer* (or esophageal cancer) is malignancy of the esophagus. There are various subtypes, primarily squamous cell cancer (approx 90-95% of all esophageal cancer worldwide) and adenocarcinoma (approx. 50-80% of all esophageal cancer in the United States). Small and localized tumors are treated surgically with curative intent. Larger tumors tend not to be operable and hence are treated with palliative care; their growth can still be delayed with chemotherapy, radiotherapy or a combination of the two. In some cases chemo- and radiotherapy can render these larger tumors operable.

*Gastric cancer,* or stomach cancer, refers to cancer arising from any part of the stomach. By the time symptoms occur, the cancer has often reached an advanced stage and may have also metastasized (spread to other, perhaps distant, parts of the body), which is one of the main reasons for its relatively poor prognosis.

### Hepatic Cancer

The compositions of the present disclosure are also useful in the treatment or prevention of hepatic cancer, with or without metastases.

*Liver cancer* or *hepatic cancer* is a cancer that originates in the liver. Liver cancers are malignant tumors that grow on the surface or inside the liver. The most frequent liver cancer is hepatocellular carcinoma (HCC) with a very poor prognosis.

### Melanoma

The compositions of the present disclosure are also useful in the treatment or prevention of melanoma.

*Melanoma* is a malignant tumor of melanocytes. Melanocytes are cells that produce the dark pigment, melanin, which is responsible for the color of skin. The treatment conventionally applied includes surgical removal of the tumor. For melanomas that come back or spread, treatments conventionally applied include chemo- and immunotherapy, or radiation therapy with poor prognosis. Also melanoma may spread and form metastases which may also be treated or prevented by the compositions of the present disclosure.

### Leukemia

The compositions of the present disclosure are also useful in the treatment or prevention of leukemia.

*Leukemia* is a type of cancer of the blood or bone marrow characterized by an abnormal increase of immature white blood cells called "blasts".

### Benign Tumor Indications

A benign tumor is a mass of cells (tumor) that lacks the ability to invade neighboring tissue or metastasize. These characteristics are required for a tumor to be defined as cancerous and therefore benign tumors are non-cancerous. Also, benign tumors generally have a slower growth rate than malignant tumors and the tumor cells are usually more differentiated (cells have normal features). Benign tumors are typically surrounded by an outer surface (fibrous sheath of connective tissue) or remain with the epithelium. Common examples of benign tumors include melanocytic nevus (nevi) and uterine fibroids (leiomyomas).

### Uterine fibroids

*Uterine fibroids* (also referred to as myoma, leiomyoma, leiomyomata, and fibromyoma) are benign (non-cancerous) tumors that grow within the muscle tissue of the uterus. Between 20-50% of women of childbearing age have uterine fibroids. While many women do not experience any problems, symptoms can be severe enough to require treatment. Myomas may grow as a single nodule or in clusters and may range in size from 1 mm to more than 20 cm in diameter. Myomas are the most frequently diagnosed tumor of the female pelvis and the most common reason for a woman to have a hysterectomy. The cause of myomas has not actually been determined. The current treatment of uterine fibroids generally involves surgery.

### Description of ingredients of the composition

In the composition according to the present disclosure, several active agents contained in natural plants and algae are used. The term "compound" as used in the present disclosure refers to a natural plant or algae as used herein, to a part of said plant or algae, to an active agent present in said plant or algae, or to a specific single compound present in said plant or algae, or also to an extract of said plant or algae. In the following, the corresponding species are illustrated in more detail.

For the purpose of the present disclosure, if "approximately equal amounts" of the compounds are used, the difference in the amounts (by volume or mass, respectively) is less than 15 %, preferably less than 10 %, more preferably less than 5 % and most preferably less than 1 % (by volume or mass, respectively).

### Cyanobacteria

Cyanobacteria, also known as blue-green bacteria, blue-green algae, or Cyanophyta, is a phylum of bacteria that obtain their energy through photosynthesis. In the present disclosure, Arthrospira or Spirulina is preferably used as cyanobacteria. Preferably, Arthrospira platensis and Arthrospira maxima are used in the disclosure. Arthrospira is a genus of free-floating filamentous cyanobacteria characterized by cylindrical, multicellular trichomes in an open left-hand helix. Spirulina is mainly commercially produced at the Hawaii Islands and in California. The FDA (US Food and Drug Authority) rewarded under sections 20 1 (s) and 409 of the Federal Food, Drug, and Cosmetics Act the Spirulina produced by Cyanotech Corporation of Hawaii and Earthrise Nutritionals LLC of California GRAS (Generally Recognized As Safe). Therefore these two producers would be preferred for the use in compositions of the present disclosure. Studies have shown that polysaccharide extracts increase macrophage function, antibody production and infection fighting T-cells. Polysaccharides and phycocyanin from Spirulina have shown to increase immunity in mice by enhancing bone marrow reproduction, growth of thymus and spleen and biosynthesis of serum protein.

In a preferred embodiment of the present disclosure, the dried *cyanobacteria* have at least 80 mg of lithium per gram of dry *cyanobacteria,* preferably Spirulina. *Cyanobacteria* are referred to in the present disclosure also as "algae", falling under said general term.

### Agaricus subrufescens

Agaricus subrufescens, also termed as Agaricus blazei or Agaricus blazei murill, Agaricus brasiliensis, or Agaricus rufotegulis, is a species of mushroom. Agaricus subrufescens was traditionally used to treat many common diseases like atherosclerosis, hepatitis, hyperlipidemia, diabetes, dermatitis and cancer. In vitro and in vivo, Agaricus subrufescens has shown immunomodulatory and antimutagenic properties. For the present disclosure, an aqueous extract of Agaricus subrufescens is preferably used, more preferably from the cap of Agaricus subrufescens, further preferred in the presence of sucrose.

### Carica papaya

The papaya is the fruit of the plant *Carica papaya,* the sole species in the genus Carica of the plant family Caricaceae. Two kinds of papayas are commonly grown. One has sweet, red (or orangish) flesh, and the other has yellow flesh. Either kind picked green, i.e., unripe, is called a "green" papaya. For the compositions used in the present disclosure, preferably the fruit of the green, i.e. unripe, papaya is used. In another preferred embodiment of the present disclosure, the *carica papaya* is used as an extract, preferably an aqueous extract, more preferably as an extract or aqueous extract of the green papaya.

### Pineapple

The *pineapple* (Ananas comosus) is a tropical plant in the Bromeliaceae family. Pineapple contains four distinct cysteine proteinases. The major proteinase present in extracts of plant stem is stem bromelain, whilst fruit bromelain is the major proteinase in the fruit. Two additional cysteine proteinases are present only in the stem: these are ananain and comosain. Stem bromelain, fruit bromelain and ananain are immunologically distinct. Bromelain is the most important enzyme.

The fruit and/or stem of *pineapple* may be used in the compositions of the present disclosure. For the compositions of the present disclosure, preferably an extract of the fruit and/or stem of *pineapple,* more preferably extracts of both fruit and stem, are used. In a further preferred embodiment, the extract is an aqueous extract.

### Hypericum perforatum

St John's wort is the plant species *hypericum perforatum,* and is also known as Tipton's weed, chase-devil, or Klamath weed. *Hypericum perforatum* is a perennial plant with extensive, creeping rhizomes. Its stems are erect, branched in the upper section, and can grow to 1 m high. For the compositions of the present disclosure, the whole plant of *hypericum perforatum,* more preferably locally grown *hypericum perforatum,* may be used. In a preferred embodiment of the present disclosure, the *hypericum perforatum* is used as an extract, preferably an aqueous extract.

### Sambucus

*Sambucus,* also referred to as elder or elderberry, is a genus of between 5 and 30 species of shrubs or small trees in the moschatel family, Adoxaceae. A specific genus is not preferred in the present disclosure. For use in the compositions of the present disclosure, the flowers (flos) of *sambucus* are preferred. For the purpose of the preparation of a liquid extract from *sambucus,* preferably the flowers (flos) is used, however, any part of the plant may be used to prepare an extract. In a further preferred embodiment, the extract is an aqueous extract.

### Agaricus campestris

Agaricus campestris is also commonly known as field mushroom or meadow mushroom. For the present disclosure, any part of *agaricus campestris* may be used, preferably the cap of *agaricus campestris.* In a preferred embodiment of the present disclosure, *agaricus campestris* is used as an extract, preferably an aqueous extract. If an extract, preferably an aqueous extract is used, the cap of *agaricus campestris* is preferably used.

### Petroselinum

Petroselinum (parsley) is a genus of two species of flowering plants in the family Apiaceae, native to western and southern Europe and northern Africa. From the two species Petroselinum crispum (Garden Parsley) and Petroselinum segetum (Corn Parsley), the leaves of Petroselinum crispum are preferred in the compositions of the present disclosure, preferably locally grown Petroselinum crispum. In a preferred embodiment, an extract, preferably an aqueous extract of *petroselinum* is used.

### Ranunculus

Ranunculus is a large genus of about 600 species of plants in the Ranunculaceae. Members of the genus include the buttercups, spearworts, water crowfoots and the lesser celandine. For the compositions of the present disclosure, the whole dried plant of ranunculus acris and ranunculus repens are preferred, more preferred as an extract, even more preferred as an aqueous extract.

### Nicotiana

Nicotiana is a genus of herbaceous plants and shrubs of the family Solanaceae, indigenous to the Americas, Australia, south west Africa and the South Pacific. Various nicotiana species, commonly referred to as tobacco plants, are cultivated as ornamental garden plants. The leaves, preferably dried, of any of the various species known may be used in connection with the compositions of the present disclosure. In a preferred embodiment, extracts of nicotiana are used, more preferably aqueous extracts. In a more preferred embodiment, extracts of the leaves, preferably of the dried leaves, are used, even more preferably aqueous extracts.

### Magnesium phosphoricum

Magnesium phosphoricum is an aqueous solution of dimagnesium phosphate (MgHPO₄). For simplification, magnesium phosphoricum is also referred to as a compound in the present disclosure, and in some occasions also falls under the term plant or algae when referring to the composition of the present disclosure. The skilled person will nevertheless understand that an extract may not be prepared from magnesium phosphoricum, but rather a solution of magnesium phosphoricum is prepared.

### Preferred combinations

Preferred combinations of the above given compounds resulting in an inventive composition according to the present disclosure are summarized in Table 1a below. As can be gathered also from Table 1a, the compositions of the present disclosure comprise at least the compounds *agaricus subrufescens, carica papaya* and *pineapple.*

Other combinations of the compounds used in the present disclosure, however, not falling under the claims of the present disclosure, are disclosed in Table 1b below.

**Table 1a: Sub-combination of preferred compositions**

| | Ingredients | | | | | | | | | | | |
|---|---|---|---|---|---|---|---|---|---|---|---|---|
| No. | Agaricus Subrufescens | Agaricus campestris | Cyanobacteria | Sambucus | Carica Papaya | Pineapple | Hypericum perforatum | Petroselinum | Ranunculus | Nicotiana | Magnesium Phosphoricum | No of ingredients |
| 1 | x | | | | | x | | | x | | | 3 |
| 2 | x | | x | | | x | | | x | | | 4 |
| 3 | x | | x | | x | x | | | x | | | 5 |
| 4 | x | | | x | | x | | | x | | x | 5 |
| 5 | x | | x | x | x | x | | | x | | | 6 |
| 6 | x | | | x | x | x | | | x | | x | 6 |
| 7 | x | | x | x | | x | | | x | | x | 6 |
| 8 | x | | | x | | x | x | | x | | x | 6 |
| 9 | x | | x | x | x | x | x | | x | | | 7 |
| 10 | x | | x | x | x | x | | | x | x | | 7 |
| 11 | x | | x | x | x | x | | x | x | x | x | 9 |
| 12 | x | | x | x | x | x | x | x | x | x | x | 10 |
| 13 | x | x | x | x | x | x | x | x | x | x | x | 11 |

**Table 1b: Sub-combination not falling under the present invention**

| | Ingredients | | | | | | | | | | | |
|---|---|---|---|---|---|---|---|---|---|---|---|---|
| No. | Agaricus Subrufescens | Agaricus campestris | Cyanobacteria | Sambucus | Carica Papaya | Pineapple | Hypericum perforatum | Petroselinum | Ranunculus | Nicotiana | Magnesium Phosphoricum | No of ingredients |
| 14 | | x | | x | x | | | | | | x | 4 |
| 15 | x | | | x | | | | | x | | x | 4 |
| 16 | x | | x | | | x | | | | x | x | 5 |
| 17 | x | | x | | x | | | | | x | x | 5 |
| 18 | x | | x | x | | | | x | x | | | 5 |
| 19 | x | | x | | | x | | x | | x | | 5 |
| 20 | | x | x | x | x | | | | | | x | 5 |
| 21 | x | | | x | x | | | | x | | x | 5 |
| 22 | x | | x | | x | x | | | | x | x | 6 |
| 23 | x | | x | x | x | | | | x | | x | 6 |
| 24 | x | | | x | x | | x | | x | | x | 6 |
| 25 | x | | | x | x | | | x | x | | x | 6 |
| 26 | x | | x | x | x | | | x | x | | x | 7 |

### Extracts of the plants and algae

According to the present disclosure, the composition comprising plants and algae is preferably prepared from extracts of the plants and algae. Especially preferred are aqueous extracts. It is understood that the concentration of active ingredients of the extracts of natural products, such as plants and algae, highly depend on the source of the natural products and the amount of extraction medium. The preferred extraction medium according to the present disclosure is distilled water.

A summary of possible concentrations of aqueous extracts is given in Table 2 below, based on the amount of extracted plant or algae (dried or fresh, as indicated). The plants or algae are extracted using water, preferably distilled water, at a temperature in the range of 20 to 60 °C, preferably 25 to 50 °C. The water is poured over the plant or algae in dried or fresh (i.e., non-dried) form, then allowed to brew or extract for an appropriate time, such as 1 minute to 72 hour, preferably 5 minutes to 120 minutes and in case of carica papaya 72 hours. Finally, the extracted plants or algae are filtered or centrifuged off to result in a filtrate containing extracts of the plant or algae.

**Table 2: Amounts of plants and algae used for preparing aqueous extraction**

| Compound | Amount used for extraction (g/100 ml distilled water) | Compound condition |
|---|---|---|
| Cyanobacteria | 1 to 70, | Dried |
| | preferably 10 to 70 | |
| Agaricus Subrufescens | 1 to 50, | Dried |
| | preferably 10 to 50 | |
| Pineapple | 10 to 180, | Fresh |
| | preferably 100 to 180 | |
| Carica papaya | 10 to 180, | Fresh |
| | preferably 100 to 180 | |
| Hypericum perforatum | 20 to 60, | Fresh or dried |
| | preferably 40 to 60 | |
| Sambucus flos | 20 to 60, | Fresh or dried |
| | preferably 40 to 60 | |
| Agaricus Campestris | 1 to 50, | Dried |
| | preferably 10 to 50 | |
| Petroselinum crispum | 10 to 60, | Fresh |
| | preferably 30 to 60 | |
| Ranunculus | 0,5 to 10, | Dried |
| | preferably 1 to 10 | |
| Nicotiana | 0,5 to 10, | Dried |
| | preferably 1 to 10 | |
| Magnesium Phosphoricum | 0,1 to 0,9 | Powder |

Alternatively, commercially available extracts of the corresponding plants and algae may be used in accordance with the present disclosure. As is obvious from the nature of magnesium phosphoricum, an extract may not be prepared, but the corresponding amount is dissolved in water to prepare a solution of magnesium phosphoricum.

In a preferred embodiment, the plants and algae of the present disclosure are extracted with aqueous extraction media, preferably water, more preferably distilled water, resulting in an aqueous extract of the plant or algae. However, also other extraction media may be used. Examples of other extraction media include, but are not limited to organic solvents, such as alcohols, preferably methanol, ethanol and propanol, acetic acid, acetic acid esters, acetyl acetone, and mixtures thereof, as well as mixtures with water.

The compositions of the present disclosure may then initiate an immune modulation, leading to the destruction and phagocytosis of the tumor cells in addition to an antiinflammatory action.

### Kits

The disclosure further provides kits that can simplify the handling and administration of a composition of the present disclosure to an animal.

In one embodiment, a kit of the disclosure comprises a unit dosage form of a composition of the present disclosure. In one embodiment, the unit dosage form comprises a first container, which can be sterile, containing an effective amount of a composition of the present disclosure and a pharmaceutically acceptable carrier or excipient. The kit can further comprise a label or printed instructions instructing the use of the composition of the present disclosure to treat or prevent cancer. The kit can further comprise a unit dosage form of a second therapeutic agent, for example, a second container containing an effective amount of the second therapeutic agent and a pharmaceutically acceptable carrier or excipient. In another embodiment, the kit comprises a container containing an effective amount of a composition of the present disclosure, an effective amount of a second therapeutic agent and a pharmaceutically acceptable vehicle, carrier, or excipient. Examples of second therapeutic agents include, but are not limited to, the below Additives 1 to 4.

Kits of the disclosure can further comprise a device that is useful for administering the unit dosage forms. Examples of such a device include, but are not limited to, a syringe, a drip bag, a patch, an inhaler, and an enema bag. The kit should not contain further chemical drugs, which could interact with the composition and therefore weaken the effectiveness of it.

The kit may additionally contain one or more of the following additives:
- Additive 1: Q4 composition
- Additive 2: Liquid colloidal silver
- Additive 3: Oxygen water
- Additive 4: Vitamins and minerals

These additives may be administered in addition to the composition of the present disclosure, but not necessarily at the same time or even mixed with it.

### Additive 1: Special Composition Q4

Additive 1 is a special composition containing compounds, such as macrominerals and trace elements, which may be beneficial to any patient suffering from a tumor. As such, Additive 1 may be helpful in treating any disease, including benign and malign tumor indications. However, as can also be gather from the list of ingredients as given in Table 3 below, Additive 1 does not contain any "drug" or "active agent" and is thus only considered as a helpful supportive dietary, however, not essential for the treatment with the compositions of the present disclosure.

**Table 3: Ingredients of Additive 1**

| No. | Ingredient | Amount [mg/liter] |
|---|---|---|
| 1 | Calcium Phosphate | 26 |
| 2 | Potassium Citrate | 38 |
| 3 | Silicon Dioxide | 9,7 |
| 4 | Sodium Chloride | 22,3 |
| 5 | Magnesium Citrate | 42 |
| 6 | Lithium Carbonate | 370 |
| 7 | Zinc Gluconate | 0,35 |
| 8 | Hypericum perforatum | 32-77 |
| 9 | Cyanobacteria or Agaricus subrufescens | 43-108 |

### Additive 2: Colloidal silver

The silver ion (Ag⁺) is bioactive and in sufficient concentration readily kills bacteria *in vitro.* Silver also kills bacteria in external wounds in living tissue, so physicians use wound dressings containing silver sulfadiazine (Ag-SD) or silver nanomaterials to treat external infections. Wound dressings containing silver are increasing in importance due to the recent increase of antibiotic-resistant bacteria, such as methicillin-resistant Staphylococcus aureus (MRSA). The disinfectant properties of silver are used in medical applications, such as urinary catheters and endotracheal breathing tubes, where the silver content is effective in reducing incidences of catheter-related urinary tract infections and ventilator-associated pneumonia (VAP), respectively. Silver is also used in bone prostheses, reconstructive orthopedic surgery and cardiac devices, as well as on surfaces and fabrics to reduce the spread of infection. The mild silver in colloidal form is used as solution.

### Additive 3: Oxygen water

Normal tab water filtered (filter type active coal filter, e.g. Carbonit Premium, with a filter pore size of 0,450 µm) enriched with oxygen with a content greater than 25 mg/l water, may be used in addition to the compositions of the present disclosure. Such oxygen enriched water is termed "oxygen water" for the purpose of the present disclosure.

For the present disclosure, if water is enriched with oxygen, the oxygen may be dissolved in water, such as physically or chemically dissolved in the water, or adhere to any of the compounds of the present disclosure.

### Additive 4: Vitamins and minerals

The treatment may also be accompanied by the application of natural vitamins and/or minerals. There is no specific kind or source of vitamin and/or mineral, but natural sources of vitamins and minerals are preferred for their usually high bioavailability. Any combination of vitamin(s) and/or mineral(s) may be used, preferably vitamin(s) and/or mineral(s) having a high bioavailability. Exemplary vitamins are C, A, D, B12 and the like, and exemplary minerals are zinc, magnesium, calcium, potassium, and the like. Dosage of any of these vitamins and minerals is preferably 100% of the recommended daily allowance (RDA).

### Application

The compositions or the present disclosure may be applied, among others, orally, intravenously and subcutaneously. Further, the composition can also be applied topically or inhaled using a vaporizer. In a preferred embodiment of the present disclosure, the compositions are applied orally or topically. Both the oral and topical application may be used to cancer systemically or locally. In other words, also a topical application may be used to treat any cancer since the active agents of the composition are resorbed via the skin and transported via the blood or lymph to the tumor. On the other hand, the compositions of the present disclosure may also be used as a local treatment for cancers close to the site of topical application if, e.g., applied topically. In a more preferred embodiment of the present disclosure, the compositions are applied orally.

Together with the compositions of the present disclosure, also additives may be applied. These additives may be co-administered with the compositions, i.e., taken together simultaneously or even admixed with the compositions. This applies to all modes of application, especially the oral and topical application. However, the additives may also be administered separately, i.e., physically separate from the compositions, and also with a time difference. If a topical application of the composition is used, a further topical application of additives, such as Additive 1, is not necessary, however, such additive may be applied in any way described above, such as topically or orally, or both.

### Dosage

The compositions of the present disclosure are preferably applied together with Additives 1 and 2. A preferred way of application is the oral application. An exemplary dosage regime for oral application of the composition and Additives 1 to 3 for an adult patient is listed in Table 4. The dosage amount may depend on the type of tumor and its severity.

**Table 4: dosage regime**

| Substance | Amount [ml] per dose | Frequency | Time of Day |
|---|---|---|---|
| Composition of the present disclosure | 1 to 5 | 3 times daily | Morning: fasting, 30 minutes before breakfast |
| | | | Noon: 30 minutes before lunch |
| | | | Afternoon: preferably not later than 17:00, before dinner |
| Additive 1 | 3 to 10 | 3 times daily | Morning: fasting, 30 minutes before breakfast |
| | | | Noon: 30 minutes before lunch |
| | | | Afternoon not later than 17:00, before dinner |
| Additive 2 | 10 to 30 | 3 times daily | Morning: fasting, 30 minutes before breakfast |
| | | | Noon: 30 minutes before lunch |
| | | | Afternoon not later than 17:00, before dinner |
| Additive 3 | 500 to 1500 | evenly distributed | Small amounts evenly distributed during the day |

### BRIEF DESCRIPTION OF THE DRAWINGS

Exemplary embodiments of the invention will be described in the following with reference to the following drawings:

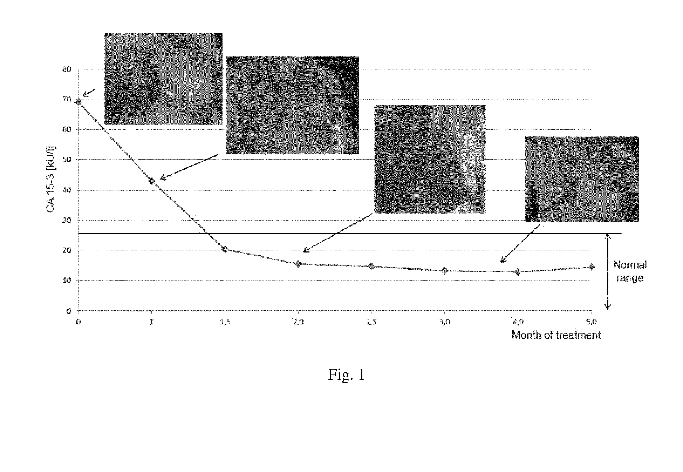

Fig. 1 showing a vast and complete remission of a mamma carcinoma pT4d including metastases of Case Report 1;

### EXPERIMENTAL SECTION

In the following, the present invention is illustrated in more detail. However, it is understood that the scope of protection is only determined by the attached claims, not being restricted to any of the following Examples. The following Examples are set forth to assist in understanding the invention and should not be construed as specifically limiting the invention described and claimed herein. Such variations of the invention, including the substitution of all equivalents now known or later developed, that would be within the purview of those skilled in the art, and changes in formulation or changes in experimental design, are to be considered to fall within the scope of the invention incorporated herein.

### In-vitro Tests

The efficacy of the compositions of the present disclosure, such as the combinations disclosed in Table 1a above, will be shown by different *in-vitro* tests and the following case report. The following *in-vitro* tests are used in accordance with the present disclosure:

### NK cell cytotoxicity assay

Natural killer (NK) cells are large granular lymphocytes composing 5 to 20% of the circulating peripheral blood monocytes (PBMCs). They play an important role in the innate immune response mainly against intracellular viruses and bacteria or malignant cells. Distinct from other killer cells, like cytotoxic T lymphocytes, NK cells are capable of performing their killing function without the need for clonal expansion and differentiation. The cytotoxic activity is, therefore, rather unspecific but also fast. With these mechanisms the NK cells provide the immune system with an efficient front line against various infections and tumor cells.

Reduced NK cell cytotoxicity has been described for many immune deficiencies, infections, and cancer. Hence, the determination of the NK cell functionality in parallel to the quantitative analysis of the NK cell population in the peripheral blood can provide important insights into the status of the patients' immune system.

The NK cell cytotoxicity assay allows quantifying the cytotoxic activity of NK cells *in-vitro* by incubating the isolated patients' PBMCs with a defined target cell line (K562) that is known to be efficiently lysed by NK cells. The leukemia cell line K562 triggers NK cell activation by lacking a primary inhibitory signal on their surface. Labeling of all K562 cells with a green fluorescent dye and of dead K562 cells with a red fluorescent dye allows the quantification of the patients' baseline NK cell activity by calculating the percentage of dead K562 cells after a defined co-incubation time with the patients' PBMCs. Additionally, immune modulators can be added during the incubation period to evaluate their potency to increase NK cell activity *in-vivo.*

### Lymphocyte proliferation assay

Many infections, autoimmune diseases, and malignant transformations induce mechanisms of immune escape, leading to a reduced number of immune cells or a reduced reactivity of the immune system. A whole mark of the activation of the cellular immune response is the induction of efficient cell proliferation.

The capacity of different subsets of lymphocytes to respond to a stimulus with proliferation can be analyzed *in-vitro.* Therefore, patient's peripheral blood mononuclear cells (PBMCs) are isolated from fresh blood and incubated under physiological condition for one week in the presence or absence of immune stimulators. Prior to analysis bromodeoxyuridine (BrdU), an analog of one of the DNA building bricks, can be added to the cultures. Staining of cells with a fluorescently labeled anti-BrdU antibody allows the relative quantification of cell proliferation by calculating the newly synthesized DNA. Furthermore, the employment of antibodies specific for lymphocyte subpopulations offers the possibility to analyze T, B, and NK lymphocytes separately.

During a clinical monitoring the capacity of patients' lymphocyte subsets to proliferate in response to an immune stimulus can be followed over time and give insides into the therapy dependent reactivation of the immune system. Furthermore, co-incubation of PBMCs from healthy controls with different concentrations of the immune stimulatory agent can provide deeper knowledge into the physiological and therapeutic mechanisms of the therapy.

### Preparation of basic composition

### Production process of Exemplary Composition A with ready to use extracts

Exemplary Composition A of the present disclosure may be prepared as follows:
75,5 ml of Agaricus subrufescens extract per 1 liter final solution are mixed with 1000 mg sucrose (disaccharide of glucose and fructose, C₁₂H₂₂O₁₁) per 1 liter final solution to form solution 1. Then the extracts given in the Table 5 below are added in the following order and amount to solution 1 with subsequently oxygen (O₂) being added by bubbling gaseous oxygen through the solution at an oxygen pressure of 200 kPa over 1 hour resulting in solution 2. The concentrations of the corresponding extract are listed in Table 6 below and may depend on the quality of the raw material for the extract.

**Table 5: Specification of composition for production**

| Nr. | Extract | Amount [ml/ll final solution] | Specification |
|---|---|---|---|
| 1 | Cyanobacteria | 94,3 | aqueous extract of dried cyanobacteria with preferably spirulina |
| 2 | Hypericum perforatum | 94,3 | aqueous extract of the whole plant of the Hypericum perforatum, preferably locally grown Hypericum perforatum |
| 3 | Carica Papaya | 94,3 | aqueous extract from the fruit of the green papaya |
| 4 | Petroselinum crispum | 94,3 | aqueous extract prepared from the leaves of preferably locally grown Petroselinum crispum |
| 5 | Pineapple | 94,3 | aqueous extract of the fruit and stem |
| 6 | Agaricus Campestris | 75,5 | aqueous extract preferably from the cap |
| 7 | Sambucus flos | 94,3 | aqueous extract preferably of the flowers (flos) |
| 8 | Ranunculus acris | 94,3 | aqueous extract of the whole plant of ranunculus acris and ranunculus repens |

**Table 6: Amount of plant and algae used for preparation of aqueous extract**

| Compound | Amount used for extraction (g/100 ml distilled water) | Compound condition |
|---|---|---|
| Cyanobacteria | 10 | Dried |
| Agaricus subrufescens | 35 | Dried |
| Pineapple | 100 | Fresh |
| Carica papaya | 100 | Fresh |
| Hypericum perforatum | 40 | Fresh |
| Sambucus flos | 40 | Fresh |
| Agaricus campestris | 5 | Dried |
| Petroselinum crispum | 30 | Fresh |
| Ranunculus acris | 1 | Dried |
| Nicotiana | 1 | Dried |
| Magnesium Phosphoricum | 0,1 | Powder |

Solution 2 is then centrifuged at 500g for 15 min resulting in a centrifugate being solution 3. As centrifuge, any professional type could be taken, e.g. Heraeus Megafuge 40. After that, oxygen (O₂) is added by bubbling gaseous oxygen through solution 3 at an oxygen pressure of 200 kPa for 30 minutes resulting in the final solution. The final solution is termed Exemplary Composition A and used in the following Case Reports. Apart from said Exemplary Composition A, also Additives 1 to 3 as described above are used in the Case Reports. It has to be mentioned, that additives 1 to 3 are not mandatory for the treatment of the above described indications.

### Production process of Exemplary Composition A with ingredients in dried_form

20 g dried powders of Spirulina and 1000 mg sucrose (disaccharide of glucose and fructose, C₁₂H₂₂O₁₁) are poured with 200 ml distilled water at 25-50 °C. The suspension is allowed to brew for 7 minutes, followed by a filtration (filter pore size in the range of 0,1 to 1 µm, type of filter e.g. MILLEX®GP, manufacturer e.g. MILLIPORE, Bedford, MA), resulting in solution 1. Oxygen (O₂) is added by bubbling gaseous oxygen through solution 1 at an oxygen pressure of 200 kPa over 1 hour resulting in solution 2.

Aqueous extracts of agaricus subrufescens, hypericum perforatum, carica papaya, pineapple, sambucus flos, petroselinum and ranunculus acris are made with the following procedure. Dried powder of the ingredient in a concentration listed in Table 6 is poured with distilled water at 25 °C. The suspension is allowed to extract for 60 to 120 minutes, followed by a filtration (filter pore size in the range of 0,1 to 10 µm, type of filter e.g. MILLEX®GP, manufacturer e.g. MILLIPORE, Bedford, MA), resulting in the corresponding aqueous extract. The aqueous extracts are mixed together in the order and amounts of Table 4, and subsequently oxygen (O₂) is added by bubbling gaseous oxygen through the solution at an oxygen pressure of 200 kPa over 1 hour resulting in the final solution. The final solution is termed Exemplary Composition A.

Exemplary suppliers for the dried powders or ready to use extracts are given in Table 7:

**Table 7: suppliers of ingredients**

| **Ingredient** | **Supplier** | **Specification** |
|---|---|---|
| Spirulina | Earthrise (California,USA) | Spirulina Natural Powder "The One Powder" |
| Agaricus Blazei Murill | Hawlik Gesundheitsprodukte, Germany | Agaricus Extract |
| Hypericum perforatum | Dixa AG St. Gallen, Schweiz | European type |
| Carica Papaya | Spira Verde GmbH, Germany, | Papaya fruit granulate with seeds |
| | or | |
| | fresh papaya from Green Field Exports, India | |
| Pineapple | Flora Apotheke, Hannover, Germany, | Anavit F3 |
| | or | |
| | fresh pineapple from Green Field Exports, India | |
| Sambucus flos | Dixa AG St. Gallen, Switzerland | European type |
| Nicotiana | Dixa AG St. Gallen, Switzerland | Dried |
| Ranunculus acris | Dixa AG St. Gallen, Switzerland | Dried |
| Petroselinum crispum | Dixa AG St. Gallen, Switzerland | Dried |
| Agaricus campestris | Dixa AG St. Gallen Switzerland | Dried |

### Selected case Reports

### Case Report 1: Complete remission of mamma carcinoma

A female, 79 year old patient was diagnosed with an inflammatory mamma carcinoma (pT4d, inflamed, tumor size 9 x 7 x 4 cm and with extension to the chest wall, HER-2-NEW+++ positive) at the right breast, which spread to the left breast as well as metastases at the spinal cord and thorax. The CT scan of thorax and abdomen showed am ample cutaneous and subcutaneous tumor infiltration at both breasts with infiltration of the muscle pectoralis right and left with oedematous infiltration of the pectoralis minor at both sides. Pathological increased lymph knots at both sides and tumor formation at both lower parts of the lung. Pleural effusion at right breast almost over the whole breast extended. Cutis thickening of 1,5 cm at both breasts. Tumor formation of 2,3 x 4,0 x 3,2 cm of the right adrenal gland. This stage of the cancer disease will normally lead to death within a very short time.

The patient was then treated with Exemplary Composition A with Additives 2 and 3 in a kit according to the dosage regimen given in Table 4. The tumor marker CA 15-3 (Carcinoma Antigen 15-3) and a CT scan of the thorax and abdomen at the end of the therapy were taken as progression parameter for judging the therapy result. Within 1,5 months of treatment, the tumor marker CA 15-3 decreased from 69 to 20 which is within the normal range. The patient had no pain within the whole course of therapy. A restaging at month five of the therapy with a CT scan of the thorax and abdomen showed a vast remission of the tumor formation at both sides as well as a complete remission of the pathological increased ambilateral lymph knots. Complete remission of the pleural effusion and of the tumor formation within the lower part of the lungs. No spread of the tumor to the abdomen organs. The patient is 13 months after the start of the therapy still alive and in good health conditions. The history and success of the treatment may also be seen from Fig. 1 below.

The present application furthermore also pertains to the following numbered embodiments:
1. A composition comprising the compounds *agaricus subrufescens, pineapple* and *ranunculus.*
2. Composition according to embodiment 1, additionally comprising the compound *cyanobacteria,* preferably *cyanobacteria spirulina.*
3. Composition according to any one of the preceding embodiments, additionally comprising at least one compound selected from the group consisting of *Carica papaya,* preferably green *carica papaya;* and *sambucus,* preferably *sambucus flos.*
4. Composition according to any one of the preceding embodiments, wherein the *ranunculus* is dried *ranunculus acris* or *ranunculus repens.*
5. Composition according to any one of the preceding embodiments, comprising at least the compounds *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos.*
6. Composition according to embodiment 5, additionally comprising the compound *nicotiana.*
7. Composition according to embodiment 6, additionally comprising the compounds *petroselium* and magnesium phosphoricum.
8. Composition according to embodiment 6, additionally comprising the compounds *hypericum perforatum* and magnesium phosphoricum.
9. Composition according to any one of the preceding embodiments, comprising at least the compounds *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos; Hypericum perforatum; petroselium; nicotiana;* and magnesium phosphoricum.
10. Composition according to any one of the preceding embodiments, wherein the compound is a plant or algae, preferably wherein the compound is an extract of said plant or algae, respectively, and more preferably an aqueous extract.
11. Composition according to any one of the preceding embodiments, wherein any of the compounds selected from the group consisting of *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos; hypericum perforatum; petroselium; nicotiana;* and magnesium phosphoricum, is present as an extract, preferably an aqueous extract, in an amount of from 50 to 300 ml, preferably from 70 to 260 ml, based on one liter of composition.
12. Composition according to any one of the preceding embodiments, wherein any of the compound selected from the group consisting of *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos; hypericum perforatum; petroselium; nicotiana;* and magnesium phosphoricum, is present as an extract, preferably an aqueous extract.
13. Composition according to any one of the preceding embodiments, wherein the composition further comprises a sugar, preferably sucrose.
14. Composition according to any one of the preceding embodiments, wherein the composition further comprises oxygen (O₂), preferably containing oxygen (O₂) in an amount of more than 23 mg/l.
15. Composition according to any one of the preceding embodiments, wherein the composition further comprises calcium phosphate, potassium citrate, silicon dioxide, sodium chloride, magnesium citrate, lithium carbonate, zinc gluconate, hypericum perforatum, and cyanobacteria or agaricus subrufescens.
16. Composition according to embodiment 15, wherein the composition further comprises colloidal silver.
17. Composition according to any one of embodiments 15 to 16, wherein the composition further comprises vitamins and/or minerals.
18. Composition according to any one of the preceding embodiments, wherein the composition is an aqueous composition.
19. Use of a composition according to any one of the preceding embodiments 1 to 18 for the treatment or prevention of a benign and malign tumor indication.
20. Use according to embodiment 19, wherein the benign and malign tumor indication is selected from the group consisting of
   K1. Cancer to the genitourinary system with or without metastases;
   K2. Mamma carcinoma with or without metastases;
   K3. Cancer to the gynecologic system with or without metastases;
   K4. Lung Cancer with or without metastases;
   K5. Tumors to the brain;
   K6. Colorectal cancer with or without metastases;
   K7. Pancreatic cancer with or without metastases;
   K8. Parathyroid carcinoma with or without metastases;
   K9. Cancer to the digestive system with or without metastases;
   K10. Hepatic Cancer with or without metastases;
   K11. Melanoma;
   K12. Leukemia; and
   K13. Benign tumor indications.
21. Use according to any one of embodiments 19 to 20, wherein the composition is administered orally, intravenously, subcutaneously, or topically, preferably orally or intravenously, and more preferably orally.
22. Use according to any one of embodiments 19 to 21, wherein the composition is administered once daily up to 10 times daily, preferably once daily up to five times daily, and more preferably three times daily.
23. Use according to any one of embodiments 19 to 22, wherein the treatment is applied for at least four weeks, preferably at least 12 weeks, and more preferably between 12 weeks and 48 weeks.
24. Use according to any one of embodiments 19 to 23, wherein the composition is co-administered together with at least one additive, which is not necessarily taken at the same time and/or mixed with the composition.
25. Use according to embodiment 24, wherein the at least one additive is selected from the group consisting of
   (a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
   (b) colloidal silver;
   (c) oxygen water, preferably containing oxygen (O₂) in an amount of more than 23 mg/l;
   (d) vitamin(s); and
   (e) mineral(s).
26. Use according to embodiment 25, wherein the composition is co-administered together with at least the additive
   (a) composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof.
27. Use according to embodiment 25, wherein the composition is co-administered together with at least the additive
   (a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof; and
   (b) colloidal silver.
28. Use according to embodiment 25, wherein the composition is co-administered together with at least the additives
   (a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof; and
   (c) oxygen water, preferably containing oxygen (O₂) in an amount of more than 23 mg/l.
29. Use according to embodiment 25, wherein the composition is co-administered together with at least the additives
   (a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
   (b) colloidal silver; and
   (c) oxygen water, preferably containing oxygen (O₂) in an amount of more than 23 mg/l.
30. Use according any one of embodiments 25 to 29, wherein the composition Q4 comprises a mixture of calcium phosphate, potassium citrate, silicon dioxide, sodium chloride, magnesium citrate, lithium carbonate, zinc gluconate, hypericum perforatum, and cyanobacteria or agaricus subrufescens.
31. Composition according to any one of embodiments 1 to 18 for use in the treatment or prevention of a benign and malign tumor indication.
32. Method for the treatment or prevention of a benign and malign tumor indication in an animal, comprising administering to an animal in need thereof, an effective amount of a composition of any one of embodiments 1 to 18.
33. Use of a composition according to any one of embodiments 1 to 18 for the preparation of a medicament.
34. Use of a composition according to any one of embodiments 1 to 18 for the preparation of a medicament for the treatment or prevention of a benign and malign tumor indication.
35. Method for the preparation of a composition according to any one of embodiments 1 to 18, wherein the composition is an aqueous composition, comprising the steps of
   (a) providing ready to use aqueous extracts of *agaricus subrufescens, pineapple* and *ranunculus,* and admixing said extracts to form a mixture;
   (b) adding sucrose to said mixture; and
   (c) optionally enriching said mixture with oxygen.
36. Kit of part comprising
   - a composition according to any one of embodiments 1 to 18; and
   - an additive comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and a combination thereof; and/or
   - oxygen water, preferably containing oxygen (O₂) in an amount of more than 23 mg/l; and/or
   - colloidal silver; and/or
   - vitamin(s); and/or
   - mineral(s); and
   - optionally instructions for a dosage regimen.

## Claims

1. A composition comprising the compounds *agaricus subrufescens, pineapple* and *ranunculus.*

2. Composition according to claim 1, comprising at least the compounds *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos.*

3. Composition according to any one of the preceding claims, comprising at least the compounds *agaricus subrufescens; cyanobacteria,* preferably *spirulina; pineapple; ranunculus,* preferably *ranunculus acris* or *ranunculus repens; carica papaya,* preferably green *carica papaya;* and *sambucus,* preferably *sambucus flos; Hypericum perforatum; petroselium; nicotiana;* and magnesium phosphoricum.

4. Composition according to any one of the preceding claims, wherein the compound is a plant or algae, preferably wherein the compound is an extract of said plant or algae, respectively, and more preferably an aqueous extract.

5. Composition according to any one of the preceding claims, wherein the composition further comprises a sugar, preferably sucrose, or oxygen (O₂), preferably containing oxygen (O₂) in an amount of more than 23 mg/l.

6. Composition according to any one of the preceding claims, wherein the composition further comprises calcium phosphate, potassium citrate, silicon dioxide, sodium chloride, magnesium citrate, lithium carbonate, zinc gluconate, hypericum perforatum, and cyanobacteria or agaricus subrufescens.

7. Composition according to claim 6, wherein the composition further comprises colloidal silver and/or vitamins and/or minerals.

8. Use of a composition according to any one of the preceding claims 1 to 7 for the treatment or prevention of a benign and malign tumor indication.

9. Use according to claim 8, wherein the benign and malign tumor indication is selected from the group consisting of
K1. Cancer to the genitourinary system with or without metastases;
K2. Mamma carcinoma with or without metastases;
K3. Cancer to the gynecologic system with or without metastases;
K4. Lung Cancer with or without metastases;
K5. Tumors to the brain;
K6. Colorectal cancer with or without metastases;
K7. Pancreatic cancer with or without metastases;
K8. Parathyroid carcinoma with or without metastases;
K9. Cancer to the digestive system with or without metastases;
K10. Hepatic Cancer with or without metastases;
K11. Melanoma;
K12. Leukemia; and
K13. Benign tumor indications.

10. Use according to any one of claims 8 to 9, wherein the composition is administered orally, intravenously, subcutaneously, or topically, preferably orally or intravenously, and more preferably orally.

11. Use according to any one of claims 8 to 10, wherein the composition is co-administered together with at least one additive, which is not necessarily taken at the same time and/or mixed with the composition.

12. Use according to claim 11, wherein the at least one additive is selected from the group consisting of
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
(b) colloidal silver;
(c) oxygen water, preferably containing oxygen (O₂) in an amount of more than 23 mg/l;
(d) vitamin(s); and
(e) mineral(s).

13. Composition according to any one of claims 1 to 7 for use in the treatment or prevention of a benign and malign tumor indication.

14. Use of a composition according to any one of claims 1 to 7 for the preparation of a medicament.

15. Method for the preparation of a composition according to any one of claims 1 to 7, wherein the composition is an aqueous composition, comprising the steps of
(a) providing ready to use aqueous extracts of *agaricus subrufescens, pineapple* and *ranunculus,* and admixing said extracts to form a mixture;
(b) adding sucrose to said mixture; and
(c) optionally enriching said mixture with oxygen.
